Europäisches Patentamt

European Patent Office

Office européen des brevets

(11) Veröffentlichungsnummer: **0 374 908 B1**

## EUROPÄISCHE PATENTSCHRIFT

(45) Veröffentlichungstag der Patentschrift: **23.03.94**

(21) Anmeldenummer: **89123614.3**

(22) Anmeldetag: **21.12.89**

(51) Int. Cl.⁵: **G01N 33/86**, C12Q 1/56, C12Q 1/37

(54) **Verfahren zur Bestimmung der Plasminogenaktivator-Aktivität in alpha-2-antiplasmin-haltigen Proben.**

(30) Priorität: **23.12.88 DE 3843422**

(43) Veröffentlichungstag der Anmeldung:
**27.06.90 Patentblatt 90/26**

(45) Bekanntmachung des Hinweises auf die
Patenterteilung:
**23.03.94 Patentblatt 94/12**

(84) Benannte Vertragsstaaten:
**AT BE CH DE ES FR GB IT LI LU NL SE**

(56) Entgegenhaltungen:

**BLOOD, Teil 58, Nr. 1, Juli 1981, Seiten
97-104, Grune & Stratton, Inc., New York, US;
L.A. MOROZ: "Mini-Plasminogen: A Mechanism for Leukocyte Modulation of Plasminogen Activation by Urokinase"**

**IDEM**

(73) Patentinhaber: **BEHRINGWERKE Aktiengesellschaft
Postfach 1140
D-35001 Marburg(DE)**

(72) Erfinder: **Stief, Thomas, Dr.
Avda. Kansas City 28
Bloque, 1. Apt. 225
E-41007 Sevilla(ES)**
Erfinder: **Heimburger, Norbert, Prof.-Dr.
Sonnenhang 10
D-3550 Marburg(DE)**

THE JOURNAL OF BIOLOGICAL CHEMISTRY, Teil 260, Nr. 22, 1985, Seiten 12106-12111, The American Society of Biological Chemists, Inc., Baltimore, MD, US; P. HOLVOET et al.: "A monoclonal antibody specific for Lys-plasminogen"

CLINICAL CHEMISTRY, Teil 32, Nr. 3, 1986, Seiten 482-485, The American Association for Clinical Chemistry, Inc., Winston, US; J. CHMIELEWSKA et al.: "Determination of Tissue Plasminogen Activator and Its "Fast" Inhibitor in Plasma"

L. Thomas (ed), Labor und Diagnose, Mediziznische Verlagsgesellschaft, Marburg 1988, S. 640/641

**Beschreibung**

Die Erfindung bezieht sich auf ein Verfahren zum Bestimmen der Aktivität von Plasminogen-Aktivatoren in Plasma oder anderen biologischen Flüssigkeiten.

Die Schlüsselenzyme der physiologischen Fibrinolyse im Säugertierblut stellen die beiden Plasminogen-Aktivatoren a) Gewebe-Plasminogen-Aktivator (t-PA) und b) urinärer Plasminogen-Aktivator (u-PA) dar. Plasminogen-Aktivatoren wandeln das inaktive Zymogen Plasminogen in die Protease Plasmin um. Plasmin degradiert unlösliches Fibrin in lösliche Fibrinspaltprodukte.

Die Aktivität dieser PA im menschlichen Blut ist von hoher diagnostischer Relevanz zur Erfassung des fibrinolytischen Potentials eines Patienten. Das fibrinolytische Potential gibt u. a. Aufschluß über mögliche zu erwartende Thromboembolien. PA kommen allerdings nur in geringen Mengen (ng/ml) im Plasma vor. Dies stellt hohe Anforderungen an Sensitivität und Spezifität des Nachweisverfahrens.

Nach dem Stand der Technik werden PA funktionell bestimmt, indem der Probe ihr natürliches Substrat, Plasminogen, und ein chromogenes Plasminsubstrat zugesetzt werden. Dabei erscheint die gemessene Plasminaktivität als direkt proportional verstärkte PA-Aktivität.

Allerdings wird das entstehende Plasmin sofort nahezu quantitativ durch ebenfalls in der Probe enthaltenes $\alpha$-Antiplasmin inhibiert, so daß nur geringfügige Plasminaktivität gemessen werden kann.

Untersuchungen von MOROZ (Blood, Bd. 58(1), 1981, S. 97 - 104) haben gezeigt, daß mini-Plasmin, das aus proteolytisch abgebautem Plasminogen entsteht, weniger durch $\alpha_2$-Antiplasmin inhibiert wird als Plasmin. Zur Proteolyse können grundsätzlich Elastase sowie Plasmin und Trypsin (J. Biol.Chem., Bd. 260 (27), 1985, S. 12106 - 12111) eingesetzt werden. MOROZ zeigt jedoch auch, daß $\alpha_2$ - Antiplasmin in Konzentrationen, die etwa der Hälfte des Normalwertes entsprechen, das sogenannte mini-Plasmin noch auf etwa 1/3 der ursprünglichen Aktivität inhibiert.

Die Genauigkeit der funktionellen PA-Bestimmung ist daher vom Eliminieren dieses Antiplasmineffektes abhängig. Die im Stand der Technik beschriebenen Verfahren zur funktionellen Bestimmung der PA setzen deshalb entweder zusätzliche Plasmatrennungsschritte voraus - wie eine Euglobulinfällung- oder erfordern ein Ansäuern und anschließende Neutralisierung durch hohe Verdünnungen der Probe. Beide Verfahren sind relativ zeitaufwendig und methodisch anspruchsvoll.

Aufgabe der vorliegenden Erfindung war es daher, ein Verfahren zur Verfügung zu stellen, mit dem die Aktivität von PA in Plasma, Serum oder anderen biologischen Flüssigkeiten schnell und zuverlässig bestimmt werden kann. Diese Aufgabe wird erfindunggemäß dadurch gelöst, daß man das zur Bestimmung der PA notwendige Plasminogen mit Elastase bei pH 7 - pH 8 oder Plasmin oder Trypsin bei pH 10 - 13 behandelt, bis ein Molekül entsteht, das nach Umwandlung von Plasmin nicht mehr durch $\alpha$-2-Antiplasmin inhibiert wird. Ein für die Überwachung dieser Reaktion geeigneter Test ist z. B. "Berichrom®-$\alpha$-2-Antiplasmin" der Behringwerke AG, Marburg, FRG. Die Einwirkung dieser Proteasen kann dergestalt erfolgen, daß diese in immobilisierter Form vorliegen (z. B. gekoppelt an Sepharose®) und somit im anschließend durchzuführenden Aktivitätstest nicht stört.

Es hat sich überraschenderweise gezeigt, daß in einem Verfahren zur Bestimmung von PA der Zusatz derart vorbehandelten Plasminogens die von alpha-2-Antiplasmin ungestörte Bestimmung der Aktivität von PA ermöglicht.

Gegenstand der Erfindung ist ein Verfahren zur Bestimmung der Plasminogenaktivator-Aktivität in einer alpha-2-Antiplasmin enthaltenden biologischen Flüssigkeit, dadurch gekennzeichnet, daß dieser Flüssigkeit proteolytisch abgebautes Plasminogen zugesetzt und das entstandene Plasmin mittels eines für Plasmin spezifischen chromogenen Substrats bestimmt wird.

Die Erfindung ist weiterhin in den Ansprüchen definiert und in den folgenden Beispielen erläutert, ohne den Erfindungsgedanken einzuengen.

Beispiel 1

Vorbehandlung von Plasminogen mit Elastase-$^R$Sepharose und Einsetzen im Plasma-PA-Aktivitätsnachweis

3 x 80 CTA-U (Committee of Thrombolytic Agents-Units) Plasminogen (Testplasminogen Behringwerke, Marburg, FRG) wurden in 7 ml 50 mM Tris/ HCl, pH 8.4 gelöst und a) auf pH 8.0, b) auf pH 12.0 eingestellt und c) unverändert gelassen. Je eine a) und b)-Lösung wurden bei einer Laufgeschwindigkeit von 30 ml/h über 1 ml Elastase-$^R$Sepharose (2.5 mg Protein/ml Gelbett, gekoppelt nach der Methode von Axen et al., Nature 214, 1302-1304, 1967) gegeben. Die Durchläufe wurden auf pH 8.4 eingestellt (Lösungen a) bis c)), auf 8 ml mit 50 mM Tris, pH 8.4 aufgefüllt und folgendermaßen in einem PA-Test eingesetzt:

50 μl humanes Citratplasma, welches unterschiedliche Mengen an alpha-2-Antiplasmin enthielt, hergestellt durch Mischung von Normalplasma mit alpha-2-Antiplasmin-Mangelplasma (immunadsorptiv zubereitet) wurden mit 200 μl obiger Plasminogenproben und 200 μl 5 IU/ml u-PA in 100 mM Tris, 100 mM NaCl, 1 % Polygelin, 0.1 % $^R$Triton X 100 5 min bei 37°C inkubiert. Anschließend wurden 500 μl 0.6 mM chromogenes Plasminsubstrat HD-Norvalyl-Cyclohexylalanyl-Lysyl-para-Nitroanilid in 480 mM NaCl zugesetzt, die Substratumsetzung nach 5 min 37°C durch Zusatz von 100 μl 8.5 M Essigsäure beendet und die entstandene Extinktion bei 405 nm bestimmt. Als Nullwertabgleich diente der gleiche Testansatz unter Zusatz von lediglich Trispuffer ohne u-PA-Gehalt.

## Tabelle 1

| Plasmagehalt an alpha-2- Antiplasmin | Zusatz von | | |
|---|---|---|---|
| | Plasminogen mit Elastase-$^R$Sepharose abgebaut | | Plasminogen unbehandelt (Kontrolle) |
| | pH 8 | pH 12 | |
| % | $A_{405nm}$/5 min (E x 1000)* | | |
| 100 | 910 ± 4 | 648 ± 5 | 107 ± 0 |
| 75 | 962 ± 1 | 676 ± 5 | 198 ± 17 |
| 50 | 1037 ± 3 | 771 ± 3 | 280 ± 1 |
| 25 | 1152 ± 2 | 860 ± 5 | 496 ± 1 |
| 0 | 1309 ± 3 | 1061 ± 4 | 749 ± 1 |

### * Mittelwerte von Doppelbestimmungen

Man erkennt, daß bei Verwendung nicht vorbehandelten Plasminogens die Extinktion (PA-Aktivitätsmessung) stark abhängig ist vom alpha-2-Antiplasmin-Gehalt der Probe. Durch proteolytische Vorbehandlung von Plasminogen mittels Elastase, vorzugsweise bei pH 8, verringert sich diese Abhängigkeit.

Wenn bei pH 12 abgebautes Plasminogen zugegeben wurde, betrug die Extinktion bei einem Antiplasmingehalt von 25 % 860 ± 5 und bei einem Antiplasmingehalt von 100 % 648 ± 5, das heißt lediglich 25 % weniger. Für die Kontrolle sind die entsprechenden Extinktionen 496 ± 1 und 107. Das ist ein Abfall um fast 80 %.

Beispiel 2

Vorbehandlung von Plasminogen mit Plasmin-$^R$Sepharose und Einsetzen im Plasma-PA-Aktivitätsnachweis

Plasminogen wurde gemäß Beispiel 1 aufgelöst, anschließend wurden a)- und b)-Präparation über 1 ml Plasminsepharose (2.5 mg Plasmin/ml Gel) gegeben und Durchläufe und c)-Lösung gemäß Beispiel 1 auf 8 ml (pH 8.4 ) aufgefüllt und im PA-Test untersucht.

## Tabelle 2

| Plasmagehalt an alpha-2-Antiplasmin | Zusatz von Plasminogen mit Plasmin-$^R$Sepharose abgebaut | |
|---|---|---|
| | pH 8 | pH 12 |
| % | $A_{405nm}$/5 min (E x 1000)* | |
| 100 | 114 ± 2 | 554 ± 9 |
| 75 | 113 ± 1 | 591 ± 1 |
| 50 | 168 ± 3 | 644 ± 2 |
| 25 | 346 ± 4 | 749 ± 3 |
| 0 | 603 ± 0 | 937 ± 5 |

* Mittelwerte aus Doppelbestimmungen

Kontrolle siehe Tabelle 1, da gleicher Versuchsansatz.

Man erkennt ebenfalls, daß die Abhängigkeit der PA-Aktivitätsmessung von der Antiplasminkonzentration vor allem bei Vorbehandlung von Plasminogen bei pH 12 mit Plasmin im Vergleich zur Kontrolle (Tab. 1) stark abgeschwächt ist.

### Patentansprüche

1. Verfahren zur Bestimmung der Plasminogenaktivator-Aktivität in einer α-2-Antiplasmin enthaltenden biologischen Flüssigkeit, wobei dieser Flüssigkeit proteolytisch abgebautes Plasminogen zugesetzt und das entstandene Plasmin mittels eines für Plasmin spezifischen chromogenen Substrats bestimmt wird dadurch gekennzeichnet,
   a) daß das Plasminogen mittels Elastase bei pH 7 - 8 abgebaut oder
   b) daß das Plasminogen mittels Plasmin oder Trypsin bei pH 10 - 13 abgebaut wird.

2. Verfahren nach Anspruch 1 dadurch gekennzeichnet, daß die Proteasen immobilisiert sind.

### Claims

1. A method for the determination of the plasminogen activator activity in a biological fluid containing α-2-antiplasmin, proteolytically degraded plasminogen being added to this fluid, and the resulting plasmin being determined by means of a chromogenic substrate specific for plasmin, which comprises degrading the plasminogen using elastase at pH 7 - 8, or degrading the plasminogen using plasmin or trypsin at pH 10 - 13.

2. The method as claimed in claim 1, wherein the proteases are immobilized.

**Revendications**

1. Procédé pour déterminer l'activité de l'activateur du plasminogène dans un liquide biologique contenant de l'$\alpha$-2-antiplasmine, en ajoutant à ce liquide du plasminogène décomposé par voie protéolytique et en dosant la plasmine formée à l'aide d'un substrat chromogène spécifique à la plasmine, caractérisé en ce que
   a) on décompose le plasminogène avec de l'élastase à un pH valant de 7 à 8,
   b) on décompose le plasminogène avec de la plasmine ou de la trypsine à un pH valant de 10 à 13.

2. Procédé selon la revendication 1, caractérisé en ce que les protéases sont immobilisées.